(19) Europäisches Patentamt
European Patent Office
Office européen des brevets

(11) Veröffentlichungsnummer: **0 207 413**
**B1**

(12) **EUROPÄISCHE PATENTSCHRIFT**

(45) Veröffentlichungstag der Patentschrift: **08.08.90**

(21) Anmeldenummer: **86108514.0**

(22) Anmeldetag: **23.06.86**

(51) Int. Cl.$^5$: **C 07 D 487/04,**
C 07 D 487/14,
C 07 D 241/40, C 08 G 64/04
// (C07D487/04, 241:00,
241:00),(C07D487/14, 241:00,
221:00)

# (54) Tetra-(hydroxyaryl)-bis-chinoxaline, ihre Herstellung und ihre Verwendung als Verzweigungsmittel für thermoplastische Polycarbonate.

(30) Priorität: **05.07.85 DE 3524054**

(43) Veröffentlichungstag der Anmeldung:
**07.01.87 Patentblatt 87/02**

(45) Bekanntmachung des Hinweises auf die Patenterteilung:
**08.08.90 Patentblatt 90/32**

(84) Benannte Vertragsstaaten:
**DE FR GB IT NL**

(56) Entgegenhaltungen:
**DD-B- 138 015**
**US-A-3 534 040**
**US-A-3 702 326**
**US-A-3 852 244**
**US-A-3 966 729**
**US-A-4 086 232**
**US-A-4 125 725**
**US-A-4 185 009**
**US-A-4 393 190**

(73) Patentinhaber: **BAYER AG**
**D-5090 Leverkusen 1 Bayerwerk (DE)**

(72) Erfinder: **Eckhardt, Volker, Dr.**
**Bodelschwinghstrasse 14**
**D-4150 Krefeld (DE)**
Erfinder: **Dicke, Hans-Rudolf, Dr.**
**Bodelschwinghstrasse 16**
**D-4150 Krefeld (DE)**
Erfinder: **Freitag, Dieter, Dr.**
**Hasenheide 10**
**D-4150 Krefeld (DE)**

**EP 0 207 413 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Gegenstand der vorliegenden Erfindung sind Bis-chinoxaline der Formel I

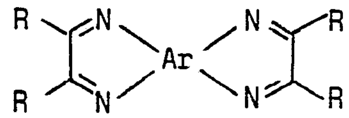

I,

worin die Reste R unabhängig voneinander 4-Hydroxyaryl- oder 3-Hydroxyaryl-Reste sind, die ausgewählt sind aus Hydroxyphenyl, Kresyl, Hydroxybiphenyl, Hydroxymethoxyphenyl, Hydroxychlorphenyl und Hydroxynaphthyl und worin Ar ein vierbindiger aromatischer Rest mit 6 bis 30 C-Atomen ist, der einkernig oder mehrkernig sein kann, wobei die mehrkernigen Reste annelliert und/oder verbrückt sein können, und wobei als Brückenglieder Heteroatome oder Carbonylgruppen fungieren.

Geeignete vierbindige aromatische Ar-Reste sind Phenylen, Naphthylen, Biphenylen, Sulfo-bis-(phenylen) Oxy-bis-(phenylen) und Carbonyl-bis-(phenylen).

Beispiele für Reste R sind 4-Hydroxyphenyl, 3-Hydroxyphenyl, 4-Hydroxy-3-methylphenyl, 4-Hydroxy-2-methylphenyl, 3-Hydroxy-5-methylphenyl, 5-Hydroxy-biphenyl(2), 4-Hydroxy-3-methoxyphenyl, 4-Hydroxy-3-chlorphenyl sowie 4-Hydroxynaphthyl(1).

Beispiele für Ar-Reste sind 1,2,4,5-Phenylen, 2,3,6,7-Naphthylen, 3,3',4,4'-Biphenylen, 2,2',3,3'-Biphenylen, Sulfo-bis-(3,4-phenylen), Sulfo-bis-(2,3-phenylen), Oxy-bis-(3,4-phenylen), Oxy-bis-(2,3-phenylen), Carbonyl-bis-(3,4-phenylen) und Carbonyl-bis-(2,3-phenylen).

Verbindungen der Formel I sind beispielsweise 2,2',3,3' - Tetra - (4 - hydroxyphenyl) - 6,6' - bis - chinoxalin, 2,2',3,3' - Tetra - (3 - hydroxyphenyl) - 6,6' - bis - chinoxalin, 2,2',3,3' - Tetra - (4 - hydroxyphenyl) - 5,5' - bis - chinoxalin, 2,2',3,3' - Tetra - (3 - hydroxyphenyl) - 5,5' - bis - chinoxalin, 6,6' - Sulfonyl - bis - (2,3 - di - [3 - hydroxyphenyl] - chinoxalin), 6,6' - Sulfonyl - bis - (2,3 - di - [3 - hydroxyphenyl] - chinoxalin), 5,5' - Sulfonyl - bis - (2,3 - di - [4 - hydroxyphenyl] - chinoxalin), 5,5' - Sulfonyl - bis - (2,3 - di - [3 - hydroxyphenyl] - chinoxalin), 6,6' - Oxy - bis - (2,3 - di - [4 - hydroxyphenyl] - chinoxalin), 6,6' - Oxy - bis - (2,3 - di - [3 - hydroxyphenyl] - chinoxalin), 5,5' - Oxy - bis - (2,3 - di - [4 - hydroxyphenyl] - chinoxalin), 5,5' - Oxy - bis - (2,3 - di - [3 - hydroxyphenyl] - chinoxalin), 6,6' - Carbonyl - bis - (2,3 - di - [4 - hydroxyphenyl] - chinoxalin), 6,6' - Carbonyl - bis - (2,3 - di - [3 - hydroxyphenyl] - chinoxalin), 5,5' - Carbonyl - bis - (2,3 - di - [4 - hydroxyphenyl] - chinoxalin), 5,5' - Carbonyl - bis - (2,3 - di - [3 - hydroxyphenyl] - chinoxalin), 2,2',3,3' - Tetra - (4 - hydroxy - 3 - methylphenyl) - 6,6' - bis - chinoxalin, 2,2',3,3' - Tetra - (4 - hydroxy - 2 - methylphenyl) - 6,6' - bis - chinoxalin, 2,2',3,3' - Tetra - (4 - hydroxy - 3 - methoxyphenyl)) - 6,6' - bis - chinoxalin, 2,2',3,3' - Tetra - (4 - hydroxy - 2 - methoxyphenyl) - 6,6' - bis - chinoxalin, 2,2',3,3' - Tetra - (4 - hydroxy - 3 - chlorphenyl) - 6,6' - bis - chinoxalin, 2,2',3,3' - Tetra - (4 - hydroxy - 2 - chlorphenyl) - 6,6' - bis - chinoxalin, 2,2',3,3' - Tetra - (6 - hydroxybiphenyl[3]) - 6,6' - bis - chinoxalin,

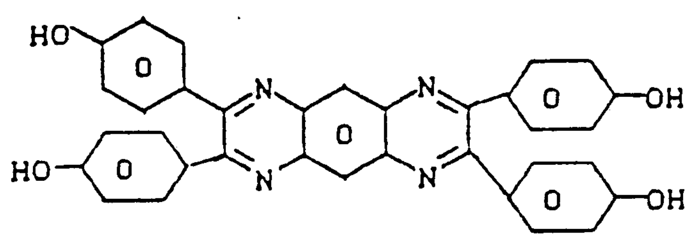

sowie

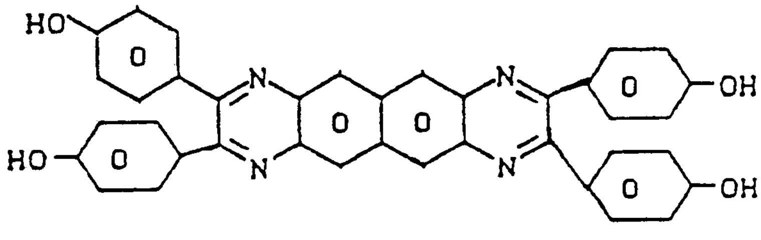

Eine bevorzugte Verbindung der Formel I ist

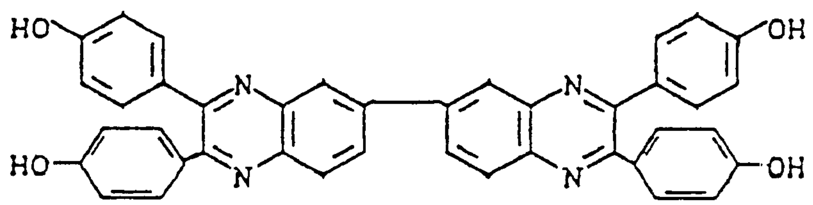

Die Herstellung der neuen Bis-chinoxaline der Formel I erfolgt durch Umsetzung der entsprechenden Tetraaminoverbindungen der Formel II mit den entsprechenden α-Diketonen der Formel III

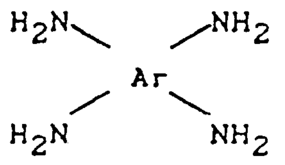 II 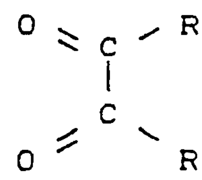 III,

worin Ar und R die für Formel I genannte Bedeutung haben, im Molverhältnis II:III wie 1:2, bei Temperaturen zwischen 20°C und 200°C, vorzugsweise zwischen 40°C und 100°C, wobei gegebenenfalls ein saurer Katalysator mitbenutzt werden kann.

Als polare Lösungsmittel dienen niedere Alkohole wie Methanol, Ethanol, Isopropanol, aliphatische Ether wie Diethylether, cyclische Ether wie Tetrahydrofuran, Dioxan, Halogenkohlenwasserstoffe wie Chloroform, Dichlormethan, 1,1-Dichlorethan oder Ester z.B. Essigsäureethylester. Das Lösungsmittel wird jeweils so gewählt, daß eine ausreichende Löslichkeit der Reaktanten, d.h. der Tetraaminoverbindung bzw. des α-Diketons gewährleistet ist.

Die Reaktion kann in Abwesenheit von Katalysatoren durchgeführt werden oder durch schwache Säuren wie Essigsäure, Propionsäure, Benzoesäure oder p-Toluolsulfonsäure katalysiert werden.

Die Reaktion wird vorzugsweise so durchgeführt, daß man das α-Diketon III in einem der vorstehend genannten Lösungsmittel löst und mit der halben molaren Menge an Tetra-amin II versetzt. Nach Zugabe eines der vorstehend genannten Katalysatoren in Mengen von 0,001—0,1 Mol-%, bezogen auf Mole II, läßt man zwischen 0,5 Stunden und 5 Stunden reagieren. Das Reaktionsprodukt fällt hierbei aus. die Isolierung kann durch Absaugen erfolgen. Eine Reinigung ist durch Lösen in Alkalien, wie verdünnter Natronlauge oder Ammoniaklösung, und nachfolgende Ausfällung in Säuren oder durch Umkristallisation aus gängigen Lösungsmitteln wie z.B. Dioxan möglich.

Gegenstand der vorliegenden Erfindung ist somit auch ein Verfahren zur Herstellung von Bis-chinoxalinen der Formel I, das dadurch gekennzeichnet ist, daß man Tetra-amine der Formel II mit Diketonen der Formel III im Molverhältnis II:III wie 1:2 in polaren organischen Lösungsmitteln bei Temperaturen zwischen 20 und 200°C über einen Zeitraum von 0,5—5 Stunden umsetzt, wobei die Reaktion gegebenenfalls durch Zusatz von 0,001—0,1 Mol%, bezogen auf Mole II, eines Katalysators beschleunigt werden kann.

Geeignete Tetraaminoverbindungen II sind beispielsweise 1,2,4,5-Tetraaminobenzol, 1,2,5,6-Tetraaminonaphthalin, 3,3',4,4'-Tetraaminodiphenylether 3,3'-Diaminobenzidin, 3,3',4,4'-Tetraaminodiphenylsulfon, 3,3',4'4-(Tetraaminobenzophenon, 2,2',3,3'-Tetraaminobiphenyl, 2,2',3,3'-Tetraaminodiphenylether, 2,2',3,3'-Tetraaminobenzophenon, 2,2',3,3'-Tetraaminodiphenylsulfon, 1,2,7,8-Tetraaminonaphthalin, 2,3,6,7-Tetraaminonaphthalin, Bevorzugte Tetraaminoverbindung ist 3,3'-Diaminobenzidin.

Die Tetraaminoverbindungen II sind entweder literaturbekannt (seihe beispielsweise WO 84/01161, S. 45—59) oder gemäß literaturbekannten Verfahren herstellbar.

Geeignete α-Diketone III sind beispielsweise 4,4'-Dihydroxybenzil, 4,4'-Dihydroxy-2,2'-dimethylbenzil, 4,4'-Dihydroxy-2,2'-dichlorbenzil, 3,3'-Dihydroxybenzil, 3,3'-Dihydroxy-5,5'-dimethylbenzil, 3,3'-Dihydroxy-5,5'-dichlorbenzil, Bis-1,2-(4-hydroxynaphthyl-1)-ethandion, Bis-1,2-(6-hydroxynaphthyl-2-)-ethandion, Bevorzugtes α-Diketon ist 4,4'-Dihydroxybenzil.

Die α-Diketone III sind ebenfalls literaturbekannt oder gemäß literaturbekannten Verfahren herstellbar.

Die erfindungsgemäßen Bis-chinoxaline I sind gut geeignete Verzweiger zur Herstellung von verzweigten Polycarbonaten. Die daraus erhältlichen verzweigten Polycarbonate haben eine hohe Standfestigkeit des Schmelzstranges (Schmelzfestigkeit), so daß sie auch für die Herstellung großvolumiger Hohlkörper nach dem Extrusionsblasverfahren geeignet sind.

Gegenstand der vorliegenden Erfindung ist somit außerdem die Verwendung der Bis-chinoxaline der Formel I zur Herstellung von verzweigten, thermoplastischen aromatischen Polycarbonaten.

Die erfindungsgemäß erhältlichen Polycarbonate sind noch in den üblichen Polycarbonatlösungsmitteln, wie etwa $CH_2Cl_2$ löslich.

Die Herstellung von verzweigten Polycarbonaten ist bekannt (siehe beispielsweise US—Re 27 682 (Le A 10 076—US—CIP—Re), DE—OS 2 113 347 (Le A 13 638), DE—OS—2 254 918 (Le A 14 711), DE—OS—2 254 917 (Le A 14 719) und DE—OS—2 500 092 (Le A 16 142).

Die Bis-chinoxaline der Formel (I) können im Prinzip nach allen drei bekannten Polycarbonatherstellungsverfahren, nach dem Phasengrenzflächenverfahren, nach dem Verfahren in homogener Lösung und nach dem Schmelzumesterungsverfahren in die thermoplastischen Polycarbonate eingebaut werden. Bevorzugtes Verfahren ist das Phasengrenzflächenverfahren.

Die mit den erfindungsgemäßen Bis-chinoxalinen der Formel (I) verzweigten Polycarbonate haben ein ausgeprägtes strukturviskoses Verhalten, so daß derselbe Polycarbonattyp für die Extrusions- und für die Spritzgießverarbeitung gut geeignet ist. Die erfindungsgemäß erhältlichen verzweigten Polycarbonate haben im Bereich der bei der Extrusions-, und Spritzgießverarbeitung in Frage kommenden Deformationsgeschwindigkeiten eine starke Abhängigkeit ihrer scheinbaren Schmelzviskosität von der jeweiligen Deformationsgeschwindigkeit. Ihre scheinbare Schmelzviskosität ist bei niedrigen Deformationsgeschwindigkeiten (Extrusion) hoch und bei hohen Deformationsgeschwindigkeiten (Spritzgießverarbeitung) niedrig.

Gegenstand der vorliegenden Erfindung ist somit außerdem ein Verfahren zur Herstellung von verzweigten, thermoplastischen, aromatischen Polycarbonaten aus Diphenolen, 0,01 bis 1 Mol-%, bezogen auf Mole Diphenole, an Verzweigungsmitteln, 0,1 bis 8,0 Mol-%, bezogen auf Mole Diphenole, an monophenolischen Kettenabbrechern und Phosgen nach den Bedingungen des Phasengrenzflächenverfahrens oder nach den Bedingungen des Verfahrens in homogener Lösung, oder aus Diphenolen, 0,01 bis 1 Mol-%, bezogen auf Mole Diphenole, an Verzweigungsmitteln, und 95 Mol-% bis 120 Mol-%, bezogen auf Mole Diphenole, an Kohlensäurediarylestern nach den Bedingungen des Schmelzumesterungsverfahrens, dadurch gekennzeichnet, daß als Verzweigungsmittel Bis-chinoxaline der Formel (I) eingesetzt werden.

Gegenstand der vorliegenden Erfindung sind außerdem die nach dem erfindungsgemäßen Verfahren erhältlichen verzweigten thermoplastischen, aromatischen Polycarbonate.

Bedingungen des Phasengrenzflächenverfahrens sind bekanntlich die Kombination von wäßrigalkalischer Phase und von organischer, mit Wasser nicht mischbarer, das entstehende Polycarbonat lösender Phase, wobei beispielsweise als Alkali NaOH und als organisches Lösungsmittel $CH_2Cl_2$ oder Chlorbenzol oder Mischungen von $CH_2Cl_2$ mit Chlorbenzol geeignet sind, Reaktionstemperaturen zwischen etwa 20°C und 80°C und die Mitverwendung von tertiären Aminkatalysatoren wie Triethylamin oder N-Ethylpiperidin oder von quartären Ammoniumsalzkatalysatoren wie Tetrabutylammoniumbromid.

Die einzusetzende Menge an Phosgen kann teilweise oder ganz durch andere, unter den Reaktionsbedingungen des Phasengrenzflächenverfahrens Carbonatgruppen bildende Verbindungen, beispielsweise durch Mono- oder Bischlorkohlensäureester der einzusetzenden Diphenole oder durch $COBr_2$ ersetzt werden.

Die Menge an Molen Phosgen bzw. zwei Halogen-CO-Gruppen-Äquivalenten, per phenolischer OH-Gruppe, liegt zwischen 45 und 90 Mol%, bevorzugt zwischen 60 und 70 Mol%.

Bedingungen des Verfahrens in homogener Phase sind bekanntlich wasserfreie Gemische von Pyridin oder anderen organischen Basen mit organischen Polycarbonatlösungsmitteln wie $CH_2Cl_2$ und Reaktionstemperaturen zwischen etwa 20°C und 80°C.

Die einzusetzende Menge an Phosgen kann, wie vorstehend beschrieben, teilweise oder ganz durch andere, unter den Reaktionsbedingungen des Verfahrens Carbonatgruppen-bildende Verbindungen substituiert werden und entspricht in etwa der beim Phasengrenzflächenverfahren.

Bei der bekannten Schmelzumesterung werden die Diphenole ebenfalls in Anwesenheit von 0,01 bis 1,0 Mol-% Verzweiger sowie eines basischen Katalysators wie Natriumbisphenolat mit den Kohlensäurediarylestern bei Temperaturen von 150 bis 300°C und unter Anlegen von Vakuum umgesetzt, wobei die bei der Umesterung anfallende Phenolkomponente gleichzeitig abdestilliert wird, wobei jedoch durch entsprechende Reaktionsführung dafür gesorgt wird, daß die zum Kettenabbruch jeweils erforderliche Menge an Monophenol von 0,1 bis 8 Mol-%, bezogen auf Mole Diphenole, in der Reaktionsschmelze bleibt und somit den separaten Zusatz an monophenolischem Kettenabbrecher erübrigt. Das anfallende Polycarbonat wird hierbei ohne Reinigung über die Schmelze isoliert.

Als Kohlensäurediarylester können beispielsweise Diphenylcarbonat, Di-(halogenphenyl)carbonate, wie Di-(chlorphenyl)-carbonat, Di-(bromphenyl)carbonat, Di-(trichlorphenyl)-carbonat, Di-(tribromphenyl)-carbonat usw. Di-(alkylphenyl)-carbonate, wie Di-(tolyl)-carbonat usw. Di-(naphtyl)-carbonat, Di-(chlornaphthyl)-carbonat, Phenyltolylcarbonat, Chlorphenylchlornaphthylcarbonat oder Mischungen eingesetzt werden.

Geeignete monophenolische Kettenabbrecher sind beispielsweise Phenol, m- und p-Methylphenol, m- und p-Ethylphenol, m- und p-Propylphenol, m- und p-Isopropylphenol, p-Bromphenol, m- und p-Butylphenol sowie p-tert.-Butylphenol, letzteres ist bevorzugt.

Geeignete Diphenole sind beispielsweise Hydrochinon, Resorcin, 4,4'-Dihydroxydiphenyl, Bis-(hydroxyphenyl)-alkane, -cycloalkane, -sulfide, -ether, -ketone, -sulfoxide oder -sulfone, ferner α,α'-Bis-(hydroxyphenyl)-diisopropylbenzol sowie die entsprechenden kernalkylierten beziehungsweise kernhalogenierten Verbindungen. Bevorzugte Diphenole sind 4,4'-Dihydroxydiphenylpropan-2,2 (Bisphenol A), Tetrachlorbisphenol A, Tetrabrombisphenol A, Tetramethylbisphenol A und Mischungen

dieser Verbindungen, Bisphenol A ist besonders bevorzugt.

Bevorzugte Mischungen bestehen aus 80—99,5 Mol-%, vorzugsweise 90—98 Mol-% Bisphenol A und 20—0,5 Mol-%, vorzugsweise 10—2 Mol% Tetrahalogenbisphenol A.

Weitere Diphenole sind beispielsweise in den US-Patenten 3 028 365 und 3 062 781 beschrieben.

Zur Herstellung der Polycarbonate durch die an sich bekannte Phasengrenzflächenkondensation wird beispielswiese das Bis-chinoxalin der Formel (I) zusammen mit der Diphenolkomponente in Natronlauge gelöst und ein nicht mit Wasser mischbares Lösungsmittel, wie z.B. Methylenchlorid, Dichlorethan oder Chlorbenzol, zugegeben. Nach Einleiten von Phosgen bei Raumtemperatur werden die erfindungsgemäßen Polycarbonate unter maximalen Einbau des Verzweigers direkt in hohen Ausbeuten aus der organischen Phase nach deren Wäsche durch Abdestillieren des Lösungsmittels oder durch Ausfällen isoliert. Die Kettenlänge der Polycarbonate wird durch Zugabe des Kettenabbrechers, eingestellt. Die Polykondensation wird zusätzlich durch tertiäre Amine beispielsweise Triethylamin beschleunigt.

Bei der ebenfalls prinzipiell bekannten Lösungskondensation phosgeniert man beispielsweise in Lösung in Abwesenheit von Wasser mit mindestens der zweifachen molaren Menge an Pyridin, bezogen auf eingesetztes Phosgen, zusammen mit einem Cosolvens wie Methylenchlorid. Das entsprechende Pyridiniumchlorid und überschüssiges Pyridin werden durch Waschen mit verdünnten Mineralsäuren entfernt und die erhaltenen Polycarbonatlösung wie üblich aufgearbeitet.

Die erfindungsgemäß erhältlichen thermoplastischen, hochmolekularen, löslichen und verzweigten Polycarbonate weisen relative Viskositäten $\eta_{rel}$ von 1,23—1,80 (gemessen an Lösungen von 0,5 g Produkt in 100 ml Methylenchlorid bei 25°C), mittlere Molekulargewichte $\overline{M}_{LS}$ (gemessen durch Lichtstreuung) von 10 000 bis 200 000 und scheinbare Schmelzviskositäten von $5 \times 10^4$ bis $10^2$ Pa.s (bei 300°C und bei Deformationsgeschwindigkeiten zwischen $1 s^{-1}$ und $5 \times 10^3 s^{-1}$) auf.

Den erfindungsgemäß erhältlichen Polycarbonaten können vor, während oder nach ihrer Herstellung die für Polycarbonate üblichen Zusatzstoffe zugegeben werden, beispeilsweise Farbstoffe, Pigmente, Entformungsmittel, Stabilisatoren gegen Feuchtigkeits-, Hitze- und UV-Einwirkung, Gleitmittel, Füllstoffe wie Glaspulver, Quarzerzeugnisse oder Graphit, Molybdänsulfid, Metallpulver, Pulver höherschmelzender Kunststoffe wie Polytetrafluorethylenpulver, natürliche Fasern, wie Baumwolle, Sisal und Asbest, ferner Glasfasern der verschiedensten Art, Metallfäden sowie während des Verweilens in der Schmelze der Polycarbonate stabile und die Polycarbonate nicht merklich schädigende Fasern.

Wegen des hervorragenden Standverhaltens des Schmelzstranges eignen sich die erfindungsgemäßen Polycarbonate besonders zur Herstellung von Hohlkörpern nach dem Blasverformungsverfahren. Die ausgezeichneten strukturviskosen Eigenschaften ermöglichen es auch, zum Beispiel Extrusionfolien mit guten mechanischen Eigenschaften und verminderter Spannungsrißkorrosion leicht zugänglich zu erhalten, die auf dem Elektro- und Automobilsektor eingesetzt werden können.

Durch Spritzgießen können Formkörper und Formteile aller Art, wie Gehäuse, Spulenkörper, Abdeckungen, Haushaltsgeräte usw. hergestellt werden.

In den Beispielen angegebene Prozentgehalte beziehen sich auf das Gewicht, sofern nicht anders angegeben. Die relativen Viskositäten $\eta_{rel}$ wurden an 0,5% igen Lösungen in Methylenchlorid bei 25°C gemessen. Die scheinbaren Schmelzviskositäten (Dimension Pascal·Sekunde (= Pa.s)) bei der jeweils angegebenen Deformationsgeschwindigkeit ($s^{-1}$) wurden bei 300°C bestimmt. Weitere Einzelheiten sind den Beispielen zu entnehmen.

## Beispiele und Vergleichsversuche

A: Herstellung von 2,2',3,3'-Tetra-(hydroxyphenyl)-6,6'-bis-chinoxalin

48,8 g 4,4'-Dihydroxybenzil werden in 300 ml Methanol gelöst und 21,4 g 3,3'-Diaminobenzidin in 700 ml Methanol zugetropft. Die Reaktionsmischung wird zunächst 30 min bei Raumtemperatur gerührt, wobei allmählich ein gelber Feststoff ausfällt. Durch zweistündiges Erhitzen im Rückfluß wird die Umsetzung vervollständigt. Das Reaktionsprodukt wird abgesaugt, aus Dioxan umkristallisiert und bei 100°C im Wasserstrahlvakuum getrocknet.

Ausbeute: 53,3 g (= 85% der Theorie)

| | | | | |
|---|---|---|---|---|
| $C_{40}H_{26}N_4O_4$ (626,67) | Ber. | C 76,7 | H 4,18 | N 8,94% |
| | Gef. | 76,6 | 4,07 | 8,98% |

Das 2,2',3,3'-Tetra-(4-hydroxyphenyl)-6,6'-bis-chinoxalin, in den nachfolgenden Beispielen als Chinoxalintetraphenol bezeichnet, entspricht der Formel

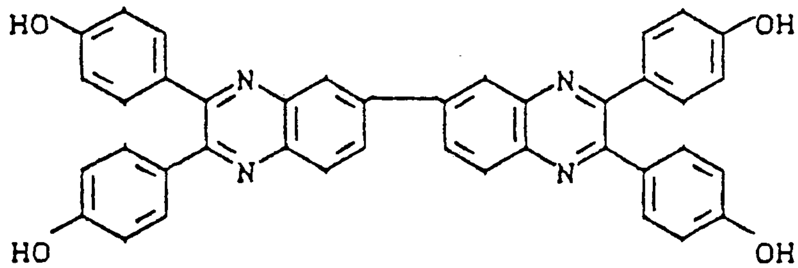

B: Verzweigte und nicht verzweigte Polycarbonate

Beispiel 1

45,6 g Bisphenol-A (BPA) und 0,125 g Chinoxalintetraphenol (0,1 mol-% Verzweiger, bezogen auf das Bisphenol A) werden unter Stickstoff in 680 g 6,2% iger Natronlauge gelöst. Eine Lösung von 0,677 g Phenol in 900 g unstabilisiertem Methylenchlorid wird zugesetzt, und in das gesamte Reaktionsgemisch unter starkem Rühren bei pH 12 bis 13 41,6 g Phosgen innerhalb 30 min. bei 25°C eingeleitet. Man gibt danach 0,2 g Triethylamin zu und läßt noch 1 h nachreagieren. Die organische Phase wird abgetrennt, zweimal mit 2% iger Phosphorsäure und dreimal bzw. solange mit Wasser gewaschen, bis die wäßrige Phase elektrolytfrei ist. Nach Abdestillieren des Lösungsmittels erhält man 43,2 g Polycarbonat mit einer relativen Viskosität: $\eta_{rel} = 1,346$.

Nach 20-minütiger Temperung der Polycarbonatschmelze bei 300°C war keine Änderung der Lösungsviskosität feststellbar. Das verzweigte Polycarbonat ist also thermische stabil.

Beispiel 2

45,6 g BPA und 0,251 g Chinoxalintetraphenol (0,2 Mol-% Verzweiger, bezogen auf Bisphenol A) werden unter Stickstoff in 680 g 6,2% iger Natronlauge gelöst und mit einer Lösung bestehend aus 0,677 g Phenol und 900 g unstabilisiertem Methylenchlorid versetzt. Unter intensivem Rühren werden innerhalb einer Stunde 41,6 g Phosgen bei Zimmertemperatur eingeleitet (pH der Lösung: 12—13). Nach Zugabe von 0,2 g Triethylamin läßt man noch 1 h weiterreagieren. Wenn die alkalische Phase frei von Bisphenol-A ist, werden die Phasen getrennt und die organische Phase zweimal mit 2% iger Phosphorsäure und dreimal mit Wasser, beziehungsweise solange gewaschen, bis kein Elektrolyt mehr im Waschwasser nachweisbar ist. Aus der organischen Phase werden nach Abdestillieren des Lösungsmittels 42,9 g verzweigtes Polycarbonat erhalten.

$\eta_{rel} = 1,378$ $M_{Vis} = 38\,700$ $M_{LS} = 105\,000$

$M_{LS}$ = Molekulargewicht, gemessen durch Lichtstreuung.

Vegleichsversuch 1

Es wurde ein Polycarbonat unter den gleichen Bedingungen wie in Beispiel 2 hergestellt, jedoch mit der Ausnahme, daß kein Chinoxalintetraphenol als Verzweiger zugegeben wurde.

$\eta_{rel} = 1,256$ $M_{Vis} = 26\,500$ $M_{LS} = 27\,000$

Beispiel 2 und Vergleichsversuch 1 verdeutlichen die hohe Verzweigung, die durch Einkondensation von Chinoxalintetraphenol in Polycarbonat erreicht wird, wie aus der Differenz der über Viskositäts- bzw. Lichtstreuungsmessung bestimmten Molekulargewichte ersichtlich ist.

Beispiel 3

22,8 g Bisphenol A, 22,7 g Diphenylcarbonat und 0,0626 g Chinoxalintetraphenol (0,1 Mol-%, bezogen auf das Bisphenol A) werden zusammen mit 0,02 mg Natriumbisphenolat in sauerstoffreier Atmosphäre aufgeschmolzen, wobei innerhalb von 5 h die Temperatur von 200°C auf 300°C gesteigert und der Druck von 100 Torr auf 1 Torr erniedrigt wird. Nach Abdestillieren des bei der Umesterung entstehenden Phenols erhält man ein transparentes Polycarbonat mit einer relativen Viskosität $\eta_{rel} = 1,247$.

Beispiel 4

Von den in Beispiel 2 sowie im Vergleichs versuch 1 hergestellten Polycarbonaten wurde zur Verdeutlichung der ausgeprägten Strukturviskosität der erfindungsgemäßen Polycarbonate die

Abhängigkeit der scheinbaren Schmelzviskosität von der Schergeschwindigkeit bei 300°C ermittelt (Düse L/D = 20).

a. Polycarbonat gemäß Beispiel 2

| | | | | | |
|---|---|---|---|---|---|
| Deformationsgeschwindigkeit ($s^{-1}$) | $10^1$ | $5 \times 10^1$ | $10^2$ | $5 \times 10^2$ | $10^3$ |
| scheinbare Schmelzviskosität (Pa.s) | 3600 | 1900 | 1400 | 700 | 500 |

b. Polycarbonat gemäß Vergleichsversuch 1

| | | | | | |
|---|---|---|---|---|---|
| Deformationsgeschwindigkeit ($s^{-1}$) | $10^1$ | $5 \times 10^1$ | $10^2$ | $5 \times 10^2$ | $10^3$ |
| scheinbare Schmelzviskosität (Pa.s) | 450 | 450 | 450 | 400 | 350 |

**Patentansprüche**

1. Bis-chinoxaline der Formel I

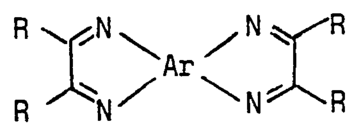

I,

worin die Reste R unabhängig voneinander 4-Hydroxyaryl- oder 3-Hydroxyaryl-Reste sind, die ausgewählt sind aus Hydroxyphenyl, Kresyl, Hydroxybiphenyl, Hydroxymethoxyphenyl, Hydroxychlorphenyl und Hydroxynaphthyl und worin Ar ein vierbindiger, aromatischer Rest mit 6 bis 30 C-Atomen ist, der einkernig oder mehrkernig sein kann, wobei die mehrkernigen reste annelliert und/oder verbrückt sein können, und wobei als Brückengleider Heteroatome oder Carbonylgruppen fungieren.

2. Bis-chinoxaline gemäß Anspruch 1, dadurch gekennzeichnet, daß Ar ein vierbindiger aromatischer Rest, ausgewählt aus Phenylen, Naphthylen, Biphenylen, Sulfo-bis-(phenylen), Oxy-bis-(phenylen) und Carbonyl-bis-(phenylen), ist.

3. Verfahren zur Herstellung der Bis-chinoxaline der Ansprüche 1 und 2, dadurch gekennzeichnet, daß man Tetraamine der Formel II

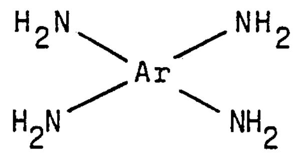

II,

worin Ar die für Formel I gemäß Anspruch 1 genannte Bedeutung hat, mit Diketonen der Formel III

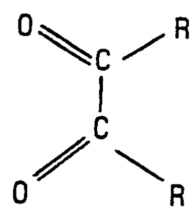

III,

worin R die für Formel I gemäß Anspruch 1 genannte Bedeutung hat, im Molverhältnis II:III = 1:2 in polaren organischen Lösungsmitteln bei Temperaturen zwischen 20 und 200°C über einen Zeitraum von 0,5—5 Stunden umsetzt, wobei die Reaktion gegebenenfalls durch Zusatz von 0,001—0,1 Mol%, bezogen auf Mole II, eines Katalysators beschleunigt werden kann.

4. Verwendung der Bis-chinoxaline der Formel I der Ansprüche 1 und 2 zur Herstellung von verzweigten thermoplastischen, aromatischen Polycarbonaten.

5. Verfahren zur Herstellung von verzweigten thermoplastischen, aromatischen Polycarbonaten aus Diphenolen, 0,01 bis 1 Mol%, bezogen auf Mole Diphenole, an Verzweigungsmitteln, 0,1 bis 8,0 Mol%, bezogen auf Mole Diphenole, an monophenolischen Kettenabbrechern und Phosgen nach den Bedingungen des Phasengrenzflächenverfahrens oder nach den Bedingungen des Verfahrens in homogener Lösung, oder aus Diphenolen, 0,01 bis 1 Mol%, bezogen auf Mole Diphenole, an

Verzweigungsmitteln und 95 Mol% bis 120 Mol%, bezogen auf Mole Diphenole, an Kohlensäurearylestern nach den Bedingungen des Schmelzumesterungsverfahrens, dadurch gekennzeichnet, daß als Verzweigungsmittel Bis-chinoxaline der Formel I der Ansprüche 1 und 2 eingesetzt werden.

6. Verzweigte, thermoplastische aromatische Polycarbonate erhältlich gemäß Verfahren des Anspruchs 5.

**Revendications**

1. Bis-quinoxalines de formule I

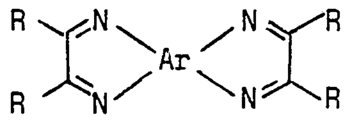

I,

dans laquelle les restes R représentent, indépendamment les uns des autres, des restes 4-hydroxyaryle ou 3-hydroxyaryle qui sont choisis parmi les restes hydroxyphényle, crésyle, hydroxybiphényle, hydroxyméthoxyphényle, hydroxychlorophényle et hydroxynaphtyle, et Ar désigne un reste aromatique tétravalent ayant 6 à 30 atomes de carbone, qui peut être monocyclique ou polycyclique, les restes polycycliques pouvant être condensés et/ou pontés, et des hétéroatomes ou des groupes carbonyle fonctionnent comme chaînons de pontage.

2. Bis-quinoxalines suivant la revendication 1, caractérisées en ce que Ar est un reste aromatique tétravalent choisi entre les restes phénylène, naphtylène, biphénylène, sulfo-bis-(phénylène), oxy-bis-(phénylène) et carbonyl-bis-(phénylène).

3. Procédé de production de bis-quinoxalines suivant les revendications 1 et 2, caractérisé en ce qu'on fait réagir des tétramines de formule II

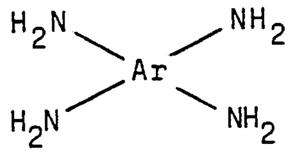

II,

dans laquelle Ar a la définition mentionnée pour la formule I suivant la revendication 1, avec des dicétones de formule III

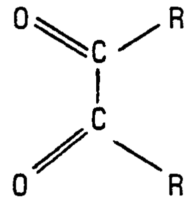

III,

dans laquelle R a la définition mentionnée pour la formule I suivant la revendication 1, dont le rapport molaire II:III est de 1:2 dans des solvants organiques polaires, à des températures comprises entre 20 et 200°C pendant une période de 0,5 à 5 heures, la réaction pouvant être accélérée le cas échéant par l'addition de 0,001 à 0,1 mole%, par rapport au nombre de moles de II, d'un catalyseur.

4. Utilisation des bis-quinoxalines de formule I suivant les revendications 1 et 2 pour la production de polycarbonates aromatiques thermoplastiques ramifiés.

5. Procédé de production de polycarbonates aromatiques thermoplastiques ramifiés à partir de diphénols, 0,01 à 1 mole%, par rapport au nombre de moles de diphénols, d'agents de ramification, 0,1 à 8,0 moles%, par rapport au nombre de moles de diphénols, d'agents monophénoliques de terminaison de chaîne et de phosgène dans les conditions du procédé à l'interface entre phases ou dans les conditions du procédé en solution homogène, ou à partir de diphénols, 0,01 à 1 mole%, par rapport au nombre de moles de diphénols, d'agents de ramification et 95 moles% à 120 moles%, par rapport au nombre de moles de diphénols, d'esters aryliques d'acide carbonique dans les conditions du procédé de transestérification à l'état fondu, caractérisé en ce qu'on utilise comme agents de ramification des bis-quinoxalines de formule I suivant les revendications 1 et 2.

6. Polycarbonates aromatiques thermoplastiques ramifiés obtenus par le procédé suivant la revendication 5.

**Claims**

1. Bis-quinoxalines corresponding to formula I

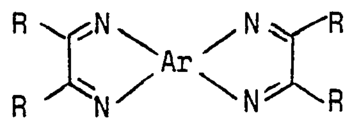

I,

in which the R's independently of one another represent 4-hydroxyaryl or 3-hydroxyaryl radicals selected from hydroxyphenyl, cresyl, hydroxybiphenyl, hydroxymethoxyphenyl, hydroxychlorophenyl and hydroxynaphthyl and in which Ar is a four-bond $C_{6-30}$ aromatic radical which may be mononuclear or polynuclear; the polynuclear radicals may be anellated and/or bridged with hetero atoms or carbonyl groups acting as bridge members.

2. Bis-quinoxalines as claimed in claim 1, characterized in that Ar is a four-bond aromatic radical selected from phenylene, naphthylene, biphenylene, sulfo-bis-(phenylene), hydroxy-bis-(phenylene) and carbonyl-bis-(phenylene).

3. A process for the preparation of the bis-quinoxalines claimed in claims 1 and 2, characterized in that tetramines corresponding to formula II

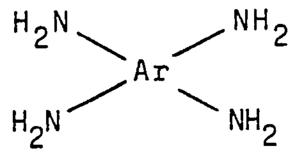

II,

in which Ar is as defined for formula I in claim 1 are reacted with diketones corresponding to formula III

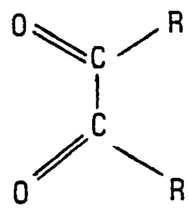

III,

in which R is as defined for formula I in claim 1, in a molar ratio of II:III = 1:2 in polar organic solvents over a period of 0.5 to 5 hours at temperatures in the range from 20 to 200°C, the reaction optionally being accelerated by addition of 0.001 to 0.1 mol-%, based on mols II, of a catalyst.

4. The use of the bis-quinoxalines corresponding to formula I in claims 1 and 2 for the production of branched thermoplastic, aromatic polycarbonates.

5. A process for the preparation of branched, thermoplastic, aromatic polycarbonates from diphenols, 0.01 to 1 mol-%, based on mols diphenols, of branching agents, and 0.1 to 8,0 mol-%, based on mols diphenols, of monophenolic chain terminators and phosgene under the conditions of the interfacial process or under the conditions of the process in homogeneous solution or from diphenols, 0.01 to 1 mol-%, based on mols diphenols, of branching agents and 95 mol-% to 120 mol-%, based on mols diphenols, of carbonic acid aryl esters under the conditions of the melt transesterification process, characterized in that bis-quinoxalines corresponding to formula I in claims 1 and 2 are used as branching agents.

6. Branched, thermoplastic aromatic polycarbonates obtainable by the process claimed in claim 5.